# EUROPEAN PATENT APPLICATION

(11) **EP 1 310 359 A2**
(43) Date of publication of application: **14.05.2003**
(21) Application number: 02024770.6
(22) Date of filing: 07.11.2002
(51) Int. Cl.: B32B 31/20

(54) **Process for making cosmetic articles**

(30) Priority: 08.11.2001 US 986265; 30.11.2001 US 996584
(71) Applicant: L'OREAL, 75008 Paris (FR)
(72) Inventor: Simon, Pascal, 94400 Vitry Sur Seine (FR)
(74) Representative: Tanty, François

(57) **Abstract**

A method of forming cosmetic articles, particularly cosmetic articles which include a surfactant such as a lathering or foaming surfactant or another cosmetic composition. In a preferred form of the method, a cosmetic composition and an adhesive are both deposited upon a substrate (10). The adhesive and/or the cosmetic composition can be provided in the form of a film or as a powder. Another substrate layer (16) is then overlaid upon the first layer, and the substrates are pressed to bond the substrate layers together. The arrangement provides a uniform product in which the surfactant composition (or other cosmetic composition) is well entrapped between the substrate layers.

## Description

The invention relates to a method of making cosmetic articles in which two substrate layers are bonded together with a cosmetic composition disposed between the substrate layers.

Articles having two substrate layers which are bonded together and which have a soap or cleansing composition disposed there between are known. For example, EP 0 353 014 discloses a wiping article which can be used for cleansing the skin or for domestic cleaning chores. In this arrangement, an adhesive is applied to portions of the substrate, the adhesive is then melted and solidified, and then a surfactant is distributed on the substrate. A second substrate also has a solidified adhesive thereon, and the second substrate is placed on the first substrate, and heat and pressure are applied to join the substrates together. The arrangement is disclosed as providing a selective attachment of the substrates at spaced positions, with surfactant particles between the attached positions. The EP '014 arrangement can be disadvantageous for a number of reasons. For example, the arrangement is somewhat complicated, requiring multiple heating steps, and the application of a surfactant between the heating steps. Moreover, the adhesive can be maintained on an exposed surface of the substrates for an extended period, which can be inconvenient and also can allow for debris to be retained by the substrate or the melted adhesive. Also, because the adhesive ultimately is disposed at irregularly spaced locations, the uniformity of the product can be poor and the most efficient use of the adhesive could also be poor. If the uniformity of the product is poor and/or an excessive amount of adhesive is utilized to ensure adequate bonding, the resulting product might be perceived as of poor quality, e.g., not uniform and/or supple throughout. If the amount of adhesive is reduced to avoid localized hard spots, the substrates might not be sufficiently adhered together. Also, because the surfactant is disposed loose between the irregularly spaced attachment locations, the surfactant can be lost through apertures in one or both of the substrates and/or the surfactant can be lost from between the substrates at the side edges of the articles.

In accordance with the invention, a process is provided in which a cosmetic composition is deposited upon a substrate layer in powder form along with an adhesive which is also in powder form. After the powders are deposited, the adhesive powder is melted, and a second substrate layer is superposed upon the first substrate layer and the substrate layers are bonded together. The adhesive can be heated to melt the powder before or after the substrate layers are superposed, or heating can be provided both before and after superposing the substrates. The invention is advantageous in providing a more uniform product which is simpler to manufacture. In particular, because the adhesive powder is mixed with the cosmetic composition powder, the surfactant and adhesive can be more uniformly distributed between the layers, and a separate heating and cooling step prior to deposition of the cosmetic composition is not needed. In addition, because the adhesive powder is melted in the presence of the cosmetic composition powder, the cosmetic composition is better retained between the substrates and loss of the cosmetic composition can be reduced. In a particularly preferred form of the invention, the adhesive and cosmetic composition powders can be pre-mixed and deposited at the same time to thereby further simplify the process. In addition, the uniformity of the product is further enhanced. According to a further preferred aspect of the invention, particles of a similar size are used for the cosmetic composition powder and the adhesive powder.

According to a further embodiment in accordance with the invention, the cosmetic composition and/or the adhesive can be provided in the form of a film which is interposed between the substrate layers. This arrangement can take a number of forms. For example, the cosmetic composition can be provided in the form of a film, with the adhesive provided as a powder such that upon heating, the adhesive powder is used to join the substrates together. Alternately, the cosmetic composition can be provided as a powder with the adhesive provided in the form of a film such that, upon heating, the adhesive film is used to join the substrates together. As a further alternative, both the cosmetic composition and the adhesive can be provided as films which are interposed between the substrate layers. As yet a further alternative, a composite film can be utilized, with the composite film having both the adhesive and the cosmetic composition such that upon heating, the adhesive can join the substrate layers together. The heating of the film or films can occur before or after the second substrate layer is superposed on the first substrate layer.

The process can be formed on a continuous or a semi-continuous basis. In particular, the substrate webs can be fed continuously such that the heating processes and joining of the webs is performed on a continuous basis. Thereafter, the joined substrate layers can be wound into a roll and thereafter cut into individual articles. Alternately, in lieu of storing the joined webs on a roll, the articles can be cut, for example, utilizing a rotary cutting arrangement so that the articles can be cut as the substrates are continuously fed. As a further alternative, a semi-continuous process can be utilized, in which the feeding of the webs is intermittently stopped to allow for cutting of the substrates into individual articles. With this semi-continuous process, the heating of the adhesive can also proceed on a semi-continuous basis, for example, utilizing a press that is timed to press the substrate layers together and heat the adhesive.

Other advantageous aspects and details of the invention are provided in the detailed description herein.

A better appreciation of the invention and many of the attendant advantages thereof will become further apparent from the following detailed description, particularly when read in conjunction with the accompanying drawing in which:
Figure 1 schematically depicts a process for making cosmetic articles according to the invention;
Figure 2 schematically depicts modifications to the process of Figure 1;
Figure 3 depicts additional modifications to the process of Figure 1; and
Figure 4 depicts a movable slack roller or dancing roller arrangement which can be utilized to allow portions of the substrate webs to be fed continuously while other portions are fed intermittently.

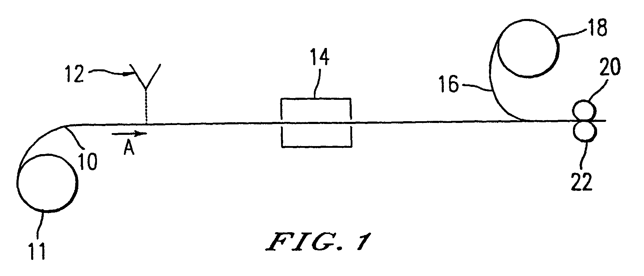

Figure 1 schematically depicts a preferred embodiment of the invention. In accordance with the preferred method, a first substrate layer 10 is fed from a supply 11 in a direction as indicated by arrow A. A mixture of an adhesive and a cosmetic composition is then deposited or sprinkled upon the substrate 10 by a suitable hopper and dispensing assembly 12, so that the mixture is deposited upon the substrate layer 10, preferably substantially uniformly over the substrate layer. The substrate layer 10 then passes through a heater 14 to melt the adhesive powder in the presence of the cosmetic composition powder. Before the adhesive is allowed to solidify, a second substrate layer 16 is overlaid upon the substrate 10, and the layers 10, 16 are then fed through a pair of nip rolls 20, 22 which are optionally heated. The nip rolls apply pressure to the superposed substrate layers 10, 16 to assist in ensuring a good bond between the substrate layers 10, 16, with the cosmetic composition entrapped between the substrate layers. If desired, additional nip rolls could be provided downstream of the nip rolls 20, 22 to further apply pressure to press or smooth the articles, and the downstream rolls could also be heated, cooled, or without temperature modification. In addition nip rolls 20, 22 could be heated as an alternative to or in addition to heating provided by heater 14. Although the amount of heat could vary depending upon, e.g., the adhesive selected, the substrate 10 and adhesive powder are preferably heated to approximately 140 C.

Because the adhesive powder is mixed with the surfactant composition before being melted to bond the substrates together, a more uniform article is provided. Also, because the adhesive and cosmetic compositions can be deposited at the same time, the process is simplified, and the product uniformity can be further enhanced because the adhesive and cosmetic composition can be more evenly distributed between the substrate layers. Also, only a single heating step is required as compared with prior arrangements in which the adhesive is applied, cooled, and then reactivated to bond the substrate layers together. Further, even where two heating steps are provided (e.g., with heating upstream and downstream of the superposing of the layers as discussed above) the top substrate layer is applied before the adhesive solidifies, and therefore the time at which a melted adhesive is exposed is reduced as compared with prior methods.

In a particularly preferred form, the cosmetic composition and adhesive are mixed before being deposited on the substrate layer 10, so that a well mixed combination of the compositions are deposited upon the substrate layer and uniformity of the product is enhanced. Alternately, the adhesive and cosmetic composition can be deposited sequentially, and uniformity of the product is nevertheless enhanced because they are mixed or commingle, since both are present in powder form before the adhesive powder is melted. As a further alternative, the cosmetic composition can be deposited on the first substrate layer after the adhesive powder is deposited and melted, with the cosmetic composition powder deposited onto the adhesive in a melted state, and with the second substrate layer superposed thereon, preferably before the adhesive has solidified. This process is also advantageous in providing good entrapment of the cosmetic composition powder because the cosmetic composition powder is deposited onto the adhesive while it is in a melted state. However, preferably the powders are mixed before being deposited upon the substrate to improve the uniformity of the product and so that the cosmetic composition is held or suspended by the adhesive (upon melting of the adhesive powder and subsequent cooling).

It is preferable to utilize adhesive and cosmetic composition powders having particles of similar size to further promote the even distribution of the adhesive and cosmetic composition upon the substrate web. Having particles of similar size is particularly preferable where they are deposited at the same time. If the particle sizes differ greatly, different sized particles could tend to settle in the hopper or dispensing device (as represented schematically at 12), which could diminish the product uniformity and result in certain articles having a greater amount of adhesive or cosmetic composition as compared with other articles. Preferably, the particles of the cosmetic composition powder and the particles of the adhesive have particle sizes of 150-700 microns. It is to be understood that other particle sizes are possible, however the particle sizes in this range are preferred. Particles having an average size of 200-500 microns are particularly preferred, with an average size of approximately 300 microns presently believed to be optimal. The powders can be provided by various known expedients. For example, the cosmetic composition can be provided by forming a solution of the composition having the desired cosmetic ingredients, and thereafter forming the composition into a dried or milled powder, the composition can be freeze-dried (lyophilization), or various granularization processes can be used. Alternately, particles of various components of the cosmetic composition can be formed, and the particles of the various components of the composition can be mixed together to form the cosmetic composition powder. However, it is presently preferred to form the desired cosmetic composition first, and then to reduce the composition to powder form. As a further alternative, a composite composition could be formed which includes both the cosmetic composition and the adhesive, with this composite composition formed into a powder, e.g., by granularization such that the granules or particles each include both the cosmetic composition and the adhesive.

The cosmetic composition can take a number of forms, including foaming compositions such as surfactants, conditioner compositions, or mixtures of surfactants and conditioners. The foregoing compositions can also include other ingredients such as vitamins, anti-wrinkle ingredients, sun screens, anti-acne, etc. The present invention could also be used for a number of other cosmetic compositions such as make-ups (e.g., a foundation make-up) or hair care compositions.

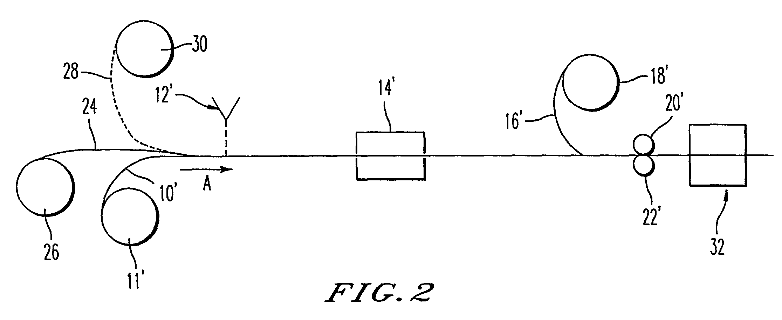

Figure 2 depicts an alternate embodiment of the process of the invention. In the arrangement of Figure 2, elements in common to those of Figure 1 are designated by primed numbers and their description will therefore not be repeated. The process represented in Figure 2 utilizes one or more films as an alternative to depositing of the adhesive and/or the cosmetic composition onto the substrate 10' in powder form. Various forms of this embodiment are possible. In particular, either the adhesive or the cosmetic composition can be provided in the form of a film 24 which is unwound from a roll 26 and superposed upon the substrate layer 10'. If the film 24 includes the adhesive, the cosmetic composition can then be deposited on top of the film 24 via hopper or other dispensing device represented at 12'. If the film 24 includes the cosmetic composition, the adhesive can then be deposited upon the film via the dispensing device 12'. Alternately, the dispensing device 12' can be disposed upstream from the location at which the film 24 is superposed upon the substrate layer 10'. However, if a film (either an adhesive film or a cosmetic composition film) is utilized in combination with a powder (e.g., for the other of the adhesive composition or the cosmetic composition), it is presently preferred to deposit the powder upon the film to minimize the possibility of the powder passing through apertures which might be present in the substrate layer 10'. If desired, the application of a cosmetic composition film or powder could also be provided downstream from the heater 14' so that the cosmetic composition is deposited onto a melted or tacky adhesive.

As a further alternative, the film 24 can be a composite of the adhesive and the cosmetic composition, in which case, the additional dispensing of a powder via dispensing device 12' is not needed. As yet a further alternative, a second film 28 can be supplied from a roll or spool 30, such that one of the films 24, 28 is provided as an adhesive film, and the other of the films 24, 28 contains the cosmetic composition. With this two film arrangement, the addition of a powder via dispensing device 12' is also not needed. Of course, depending upon the product, even if two films are provided, the addition of a powder adhesive and/or cosmetic composition could additionally be provided, e.g., to supplement the bonding provided by the adhesive film and/or to supplement the cosmetic composition with additional cosmetic composition which can be the same or different than the composition in the film. Where two films are utilized, it can be desirable to utilize additional adhesive (e.g., as a powder or an additional film) so that bonding of the substrates is ensured and a desirable bond is not blocked by the cosmetic composition film. In this regard, it is to be understood that various numbers of film layers are possible. For example, two adhesive films could be utilized with the cosmetic composition film (or powder) disposed between adhesive films. It is also to be understood that the films can be continuous or discontinuous, e.g., with apertures extending through one or more of the films. For example, where a cosmetic composition film is disposed between two adhesive films, one or both of the adhesive films could be apertured to ensure that the adhesive films do not isolate the cosmetic composition film and the cosmetic composition can be wetted when water is applied to use the article. As a further example, if a cosmetic composition film and an adhesive film are utilized, it can be advantageous to provide an apertured cosmetic composition film so that the adhesive film can bond the substrate layers together through the cosmetic composition film. The desirability or necessity to use one or more apertured films will depend upon the amount of adhesive and cosmetic composition to be used, and can also depend upon the characteristics of the substrate layers being joined (e.g., some substrates are more easily bondable than others, and certain substrates such as more porous substrates are more likely to cause a continuous adhesive and/or cosmetic composition film to become discontinuous upon heating). The desirability or need for apertures in the films can also depend upon the composition of the films. For example, certain binders for the cosmetic composition film can act as an adhesive when heated which would make the use of apertures in the cosmetic composition film unnecessary.

As with the Figure 1 embodiment, once the adhesive and cosmetic composition are deposited on the substrate layer 10', the adhesive can then be heated as the webs pass through heating device 14'. Alternately, the heat can be applied after the second substrate layer 16' is superposed upon the first substrate layer with the adhesive and cosmetic composition disposed between the first and second substrate layers 10', 16'. As a further alternative, heat can be applied both before and after superposing of the layers 10', 16'. Downstream from the rollers 20', 22', a module 32 is provided for cutting and other processing (e.g., folding) of the articles. The cutting can be performed on a continuous basis, for example, utilizing a rotary cutting device in which blades are mounted upon rollers through which the substrate layers pass after they have been bonded together. It is to be understood that this cutting operation need not be performed in-line with the other operations, and if desired, the substrate layers 10', 16' could be wound upon a roll after they are bonded together so that the substrates are stored upon the roll for further processing (e.g., cutting, folding, packaging) at another location.

The films can be formed by providing a solution of the adhesive and/or the cosmetic composition, which is then dried, for example, as the films are fed upon a large roll or upon a conveyor. Alternately, the adhesive and/or cosmetic composition can be provided as a melt mixture which is then solidified into a film upon passing over a roller or other conveyor expedient to allow the adhesive and/or cosmetic composition to cool. The adhesive can include a variety of materials including, by way of example only, ethylene vinyl ascetate or polyvinyl ascetate. The cosmetic composition film will include the desired cosmetic composition as well as a binder material so that the cosmetic composition can be provided in the form of a solution or as a heated mixture which solidifies upon drying and/or cooling upon a roll or conveyor to form the film. Suitable binders can include various polymers or other binder materials. Such binder materials could assist in bonding the substrate layers 10', 16' together in addition to the bonding provided by the adhesive film. Where the adhesive and cosmetic composition are provided as a single film, the adhesive can at least partially act as a binder for the cosmetic composition, and additional binders can be utilized if needed. It is to be understood that various types of binders and adhesives can be used as long as the cosmetic composition does not become fully isolated by the binder or adhesive such that the cosmetic composition can be wetted for use of the article. Preferably, the cosmetic composition is at least partially water soluble so that the articles can be used when wetted by water. However, cosmetic compositions could also be used which are oil soluble or which are best dissolved with an alcohol solution. The binder material could also be provided as a soluble or partially soluble material to further enhance the ability to wet the cosmetic composition for use. If apertures are desired in the adhesive and/or cosmetic composition film, they can be provided in any of various ways, e.g., utilizing apertures or projections on the surface of the support on which the film is formed, by blowing air through the film during forming of the film, or by piercing the film after it is formed.

The provision of one or both of the adhesive and cosmetic composition in the form of a film or films can be advantageous in a number or respects. For example, if both the adhesive and the cosmetic composition are provided in the form of a film or plural films, the dispensing mechanism 12' is not needed, thereby also eliminating problems which can be associated with the deposition of powders or small particulate materials (e.g., the possible loss or waste of such powder or particulate materials during deposition or subsequent handling of the articles). In addition, by utilizing one or more films, substrates having larger pores or openings therein can be utilized, since the substrate need not retain a powder or particulate composition. Even if either the adhesive or the cosmetic composition is to be provided in the form of a powder, by dispensing the powder upon the film 24, the risk of loss of the powder through the substrate layer 10' is reduced. The use of films can also be advantageous in providing a more uniform distribution of the adhesive and/or the cosmetic composition between the substrates as compared with distributing a powder upon a moving substrate, which could be subject to variations in the feed speed of the substrate and/or variations in the dispensing capabilities of the dispensing device 12'. Further, the use of films can be advantageous in that it lends itself conveniently to the ability to provide the adhesive and/or cosmetic composition at only desired locations of the completed cosmetic article. For example, if it is desired to only provide the cosmetic composition in a central region of the article, but to have the adhesive extend substantially the entire width of the article, an adhesive film 24 can be provided of a width substantially the same as the width of the substrates 10', 16', while the cosmetic composition can be provided as a film 28 having a width corresponding to the region of the article in which it is desired to have the cosmetic composition. Alternately, the cosmetic composition could be provided, for example, on only one half of the article by feeding a cosmetic composition film 28 and superposing the layer 28 on only one side of the layer 10'. Thus, viewing the substrate layer 10' in the direction of arrow A, the film 28 could be provided on the left side, on the right side, in the center, or over substantially the entire width of the substrate as desired. As a further alternative, if two cosmetic compositions are to be provided, a film containing one of the cosmetic compositions can be superposed upon the left side of the substrate layer (or the adhesive layer 24) while the right side can be provided with a film having the other cosmetic composition. As should be apparent to those skilled in the art, the use of films can be advantageous in a number of respects, including allowing for a more uniform distribution of the adhesive or cosmetic composition, or alternately, for better controlling the deposition of the adhesive or cosmetic composition to only selected regions of the substrate as desired.

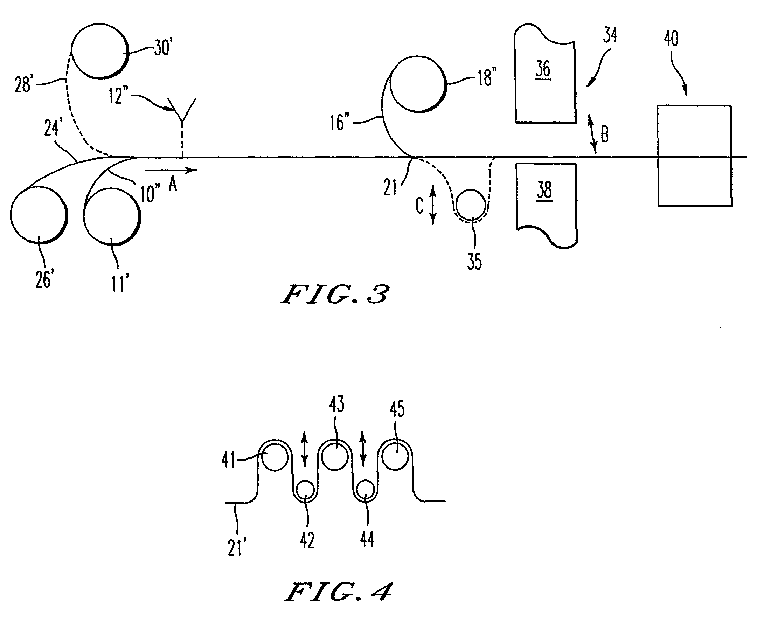

With the arrangement of Figures 1 and 2, the process can proceed continuously with the substrates and films (and/or application of one or more powders) proceeding continuously as the substrate layers are fed, superposed, heated, pressed, and cut (or wound upon a roll). Figure 3 depicts a further modification to the processes described above with reference to Figures 1 and 2. In the Figure 3 modification, the webs are intermittently halted so that the substrate layers 11", 16" can be joined by a heated press assembly 34. The heated press can include a movable hot press 36 and a stationary anvil 38, with the movable press 36 reciprocating as denoted by arrow B. Alternately, both of the press members 36, 38 could be reciprocating in timed relation, to press the substrate layers 11", 16" together and heat the adhesive to bond the substrate layers together. One or both of the press members 36, 38 can be heated. With the Figure 3 semi-continuous process, the adhesive and cosmetic composition can be deposited in various forms as discussed earlier. In particular, the adhesive or the cosmetic composition can be deposited as powders, utilizing either separate dispensing devices 12" for the adhesive and the cosmetic composition, or utilizing a single dispensing mechanism to dispense a powder containing both the adhesive and the cosmetic composition. Alternately, a film 24' can be utilized for the adhesive or the cosmetic composition, with the other of the adhesive or cosmetic composition deposited as a powder utilizing dispensing device 12". As discussed earlier, as a further alternative, the dispensing of a powder can be eliminated by providing two films 24', 28' respectively for the adhesive and cosmetic composition, or by providing a composite film 24' which includes both the adhesive and the cosmetic composition.

In the Figure 3 method, after the adhesive and cosmetic composition are deposited upon the first substrate layer 10", the second substrate layer 16" is superposed upon the first layer to form a composite web 21, with the layers of the web 21 then bonded together utilizing the heated press assembly 34. Downstream from the heated press 34 is a further processing module schematically represented at 40. As discussed earlier with reference to module 32, this processing module includes equipment to cut the composite web 21 into individual sized articles, and can include additional processing equipment, such as an assembly for folding of the articles. Because the arrangement of Figure 3 operates intermittently, with the feeding of the substrate layers halted during the pressing operation, the cutting can also be performed on an intermittent basis so that the cutting occurs while the feed of the substrate layers is halted to perform a pressing operation. It is to be understood that various feed rollers (not shown) are provided to feed the substrate layers in the processes of the invention.

If desired, one or more slack rollers or dancing rollers can be utilized to vary the amount or length of the substrate which is provided between different components so that the feed from, for example, the substrate supply rolls 11", 18" can proceed continuously despite intermittent stoppage of the composite substrate web 21 for a heating/pressing operation. For example, between the location at which the layers 10", 16" are superposed and the location 34 at which the composite web 21 is pressed, one or more slack or dancing rollers can optionally be provided with one such roller represented at 35. The dancing or slack roller is movable as represented by arrow C to vary the length of the feed path between the location at which the layers are superposed and the location of the press. With this arrangement, the slack roller moves downwardly to take up the slack caused by continued feeding of the substrates from the supply rolls 11", 18" while the composite web 21 is halted at the pressing station 34. The slack is then reduced by upward movement of the roller 35 when the press 34 is retracted and the web is fed therethrough. This use of slack or dancing rollers can be advantageous, for example, where the supply rolls or spools 11", 18" for the layers 10", 16" are large and cumbersome to controllably start and stop on a rapid intermittent basis. Thus, the feeding of the substrate layers upstream of the slack roller(s) can proceed continuously, while the web can nevertheless be intermittently stopped downstream of the slack roller(s) to perform the pressing or other processing operations.

Figure 4 depicts an alternate slack or movable roller arrangement in which five rollers 41-45 are provided, with two of the rollers 42, 44 movable. It is to be understood that various configurations and numbers of rollers are possible. With the arrangement as shown in Figure 4, the rollers act as a reservoir taking in the substrate web as the rollers 42, 44 move downwardly so that the downstream portion of the web 21' (the portion to the right of the rollers) can be halted while a pressing operation is performed even though feeding of the substrate web 21' into the rollers 41-45 continues. The reservoir can be diminished or depleted after the pressing operation is completed by upward movement of the rollers 42, 44.

In the previously described processes, the adhesive should be present in sufficient quantities to provide a good bond between the substrate layers. However, if an excessive amount of adhesive is provided, the resulting article can become stiff or less supple. In accordance with the present invention, the adhesive should be provided in an amount of at least 10 g/m2, preferably 15-50 g/m2 of the area of the substrate layer 10, 10', 10". More preferably, the adhesive should be present in an amount of 15-30 g/m2 of the first substrate layer 10, 10', 10" (which typically will also correspond to the area of the final article). Also, if the cosmetic composition is provided in insufficient quantities, it will typically not be effective, particularly if excessive amounts of adhesive isolate the cosmetic composition. Further, if an excessive amount of the cosmetic composition is provided, it can inhibit bonding between the substrates or otherwise result in an undesirable product. The cosmetic composition is preferably present in an amount of 10-100 g/m2 of the substrate layer 10, 10', 10" and more preferably 30-50 g/m2. Thus, the ratios of acceptable cosmetic composition to adhesive is preferably 1:5 to 10:1 by weight. However, it is preferable to have the amount of the cosmetic composition present in greater quantities than that of the adhesive, preferably with the cosmetic composition present in an amount from one to four times the amount of the adhesive by weight. This range is particularly preferable for foaming or lathering cosmetic compositions so that the article retains good foaming properties despite the fact that it has been disposed between a pair of adhesively bonded substrates. The amount of the cosmetic composition and adhesive could also vary depending upon the composition, the adhesive, and the substrates, however, the foregoing ranges are presently preferred. As noted earlier, where one or more films are utilized either an adhesive film and/or a cosmetic film can be provided with apertures so that a wider range of adhesive or cosmetic composition weight densities are possible while nevertheless ensuring a good bond between the substrate layers and access to the cosmetic composition when the completed article is wetted for use.

For a cleansing wipe which is to include a surfactant composition (which can include a surfactant as well as a surfactant and other ingredients, such as conditioners, vitamins, etc.), the amount of composition could be provided in a lower amount for a normal to dry skin wipe, for example, with the composition present in an amount of 15-30 g/m2, and preferably 30 g/m2. For an oily skin wipe, a higher amount of the surfactant composition can be provided, for example, 45-55 g/m2. Although not to be construed as limiting, an EVA (ethylene vinyl acetate) adhesive is presently preferred. As noted earlier, the invention is particularly preferable with surfactant compositions, for example, compositions in which at least 80% of the composition is a foaming surfactant. For cosmetic composition powders, the remainder of the powder can be fillers or other compositions, such as vitamins, conditioners, or compositions which enhance the foaming properties of the composition. However, preferably the non-surfactant ingredients are less than 20% of the total composition of the powder, and more preferably, the non-surfactant ingredients are 5-10% of the composition where the cosmetic composition is a surfactant composition for a cleansing article. Similarly, with surfactant compositions provided in a film, the composition can include a surfactant as well as additional ingredients or fillers such as vitamins, conditioners, foam enhancing compositions, etc.

It is to be understood that the method of the present invention is applicable for various articles having various substrates. Although the substrate layers are shown as separate webs 10, 16 in Figures 1-3, it would also be possible to provide the layers from a single web, for example, by folding over a single web to form the two-layered article. It is also to be understood that the invention could be utilized for articles having more than two layers or substrate plies. The method is applicable to various types of substrates and various materials, including polyamide, polyester, cellulose, rayon, polypropylene, etc. Preferably, at least 30% of the substrate will include hydrophilic fibers. However, the invention is not limited to such substrates. Further, the invention could be utilized with various foam or sponge-like materials or with various natural cloth products if desired. A wide variety of non-woven substrates are available which can be utilized. Various nonlimiting examples of such substrates include Duralace 7006, Duralace 7123, Duralace 9796 (produced by PGI), Sontara 8021, Sontara 8801, Sontara 9951, Sontara 9957, (with the Sontara line available from DuPont), Norafin 1.73065.01, Aquadim VE 50 L (from Tharreau), Lifast 55, ref 321055 (from Jacob-Holm), BBA Ultraloft 182-010, Flexilon 140-130, and Novonette 149-807. If desired, two different substrate layers can be provided so that one of the substrate layers has different properties than the other.

As should be readily apparent, the invention provides an advantageous method for forming cosmetic articles in which a cosmetic composition is disposed between substrate layers of the article. The arrangement is advantageous as compared with prior methods in that the method is relatively simple, yet results in a reliable and uniform product. Where the adhesive and/or the cosmetic composition is provided as a powder, the arrangement of the invention is also advantageous in that, because the cosmetic composition is mixed with the adhesive powder, the cosmetic composition is better held between the substrates until use of the article is desired, and the cosmetic composition powder can be suspended or partially suspended in the adhesive. Alternately, where the adhesive and/or cosmetic composition is provided as a film, greater uniformity (or if desired, application to only desired selected areas) can be achieved with lower loss/waste of the adhesive or cosmetic composition, and moreover, the films can be used with a wider variety of substrates including substrates with large apertures. By contrast, with prior methods which attach the substrates at selective irregular locations with loose powder between the attached locations, the powder can be lost either through side edges of the article or through apertures or pores within the substrates.

The terms "cosmetic articles" and "cosmetic composition" refer to products as defined in the Council Directive 93/35/EEC of 14 June 1993.

Obviously, numerous modifications and variations of the present invention are possible in light of the above teachings. It is therefore to be understood that, within the scope of the appended claims, the invention may be practiced otherwise than as specifically described herein.

## Claims

1. A method of making a cosmetic article comprising:
providing an adhesive wherein said adhesive is in the form of one of a powder and a film;
providing a cosmetic composition wherein said cosmetic composition is in the form of one of a powder and a film;
depositing said adhesive and said cosmetic composition on a first substrate layer (10);
superposing a second substrate layer (16) upon said first substrate layer (10) after depositing said adhesive and said cosmetic composition thereon;
applying heat to melt said adhesive such that said second substrate layer (16) is bonded to said first substrate layer (10) upon solidification of said adhesive.

2. A method according to the preceding claim, wherein the step of applying heat includes applying heat to melt said adhesive before said second substrate layer (16) is superposed upon said first substrate layer (10) and wherein said second substrate layer is superposed upon said first substrate layer before said adhesive solidifies.

3. A method according to any one of the two preceding claims, further including providing one of said adhesive and said cosmetic composition in the form of a powder and providing the other of said adhesive and said cosmetic composition in the form of a film (24).

4. A method according to the preceding claim, wherein said adhesive is provided in a film (24).

5. A method according to the preceding claim, wherein said film (24) containing said adhesive is superposed on said first substrate layer (10), and thereafter the cosmetic composition powder is deposited on said film (24).

6. A method according to claim 3, wherein said cosmetic composition is provided in a film (24).

7. A method according to the preceding claim, wherein said film (24) containing said cosmetic composition is superposed on said first substrate layer (10), and thereafter the adhesive powder is deposited on said film (24).

8. A method according to any one of claims 1 or 2, further comprising providing said adhesive and said cosmetic composition in the form of a film (24) containing both said adhesive and said cosmetic composition.

9. A method according to any one of claims 3 to 8, wherein said film (24) includes a plurality of apertures.

10. A method according to any one of claims 1 or 2, further comprising providing said adhesive in a first film (24, 28) and providing said cosmetic composition in a second film (24, 28).

11. A method according to the preceding claim, wherein at least one of said first film and said second film includes a plurality of apertures.

12. A method according to any one of claims 1 or 2, providing said cosmetic composition in the form of a powder and said adhesive in the form of a powder.

13. A method according to the preceding claim, wherein the step of depositing said adhesive powder and said cosmetic composition powder comprises providing a mixture of said adhesive powder and said cosmetic composition powder, and depositing said mixture on said first substrate layer.

14. A method according to any one of the two preceding claims, wherein said cosmetic composition powder includes particles having an average size of 150-700 µm, and said adhesive powder includes particles having an average size of 150-700 µm.

15. A method according to any one of claims 12 or 13, wherein said cosmetic composition powder includes particles having an average size of 200-500 µm, and said adhesive powder includes particles having an average size of 200-500 µm.

16. A method according to any one of claims 12 to 15, wherein said second substrate layer (16) is bonded to said first substrate layer (10) upon cooling of said melted adhesive.

17. A method according to any one of claims 12 to 16, wherein said cosmetic composition powder and said adhesive powder are both deposited upon said first substrate layer (10) before the adhesive powder is melted.

18. A method according to any one of claims 1, 2 or 12 to 17, wherein said adhesive and said cosmetic composition are provided as a composite powder containing both said adhesive and said cosmetic composition, and wherein said powder has an average particle size of 150-700 µm, and further wherein the step of depositing said adhesive and said cosmetic composition on said first substrate layer includes depositing said composite powder on said first substrate layer to simultaneously deposit said adhesive and said cosmetic composition on said first substrate layer.

19. A method according to any one of the preceding claims, wherein the step of applying heat includes applying heat after said second substrate layer (16) is superposed upon said first substrate layer (10).

20. A method according to any one of the preceding claims, wherein said cosmetic composition is provided in an amount of 30-50 g/m² of said first substrate layer (10).

21. A method according to any one of the preceding claims, wherein said adhesive is provided in an amount of 15-30 g/m² of said first substrate layer (10).

22. A method according to any one of the preceding claims, wherein a weight ratio of the cosmetic composition to said adhesive is in the range of 1:5 to 10:1.

23. A method according to any one of claims 1 to 22, wherein a weight ratio of the cosmetic composition to said adhesive is greater than 1:1.

24. A method according to any one of the preceding claims, wherein said cosmetic composition is deposited after the adhesive is melted and before the melted adhesive solidifies.

25. A method according to any one of the preceding claims, wherein said first substrate layer (10) and said second substrate layer (16) are fed while said cosmetic composition and said adhesive are deposited on said first substrate layer (10).

26. A method according to the preceding claim, further including halting feeding of said first and second substrate layers during heating of said adhesive.

27. A method according to the preceding claim, wherein said heating of said adhesive comprises pressing said first and second substrate layers together in a heated press (34) after said second substrate layer (16) is superposed on said first substrate layer (10) with said adhesive and said cosmetic composition disposed between said first substrate layer and said second substrate layer.

28. A method according to the preceding claim, wherein a composite web (21) is formed by said first and second substrate layers with said adhesive and cosmetic composition disposed therebetween, and wherein the method further includes cutting said composite web (21) at a location downstream from said heated press (34).

29. A method according to the preceding claim, wherein said cutting is performed while feeding of said first and second substrate layers is halted to perform a heating step to bond the substrate layers together.

30. A method according to any one of the preceding claims, wherein said first and second substrate layers are fed from respective first (11) and second (18) supply rolls, and wherein after said second substrate layer is superposed on said first substrate layer, the first and second substrate layers are fed to at least one movable slack roller (35) which allows intermittent feeding of the first and second layers downstream of the at least one movable slack roller while feeding of the first and second substrate layers from the first and second supply rolls to the at least one slack roller can proceed continuously.

31. A method of making a cosmetic article comprising:
providing a powder which includes an adhesive and a cosmetic composition;
depositing said powder onto a first substrate layer;
applying heat to melt said adhesive; and
superposing a second substrate layer upon said first substrate layer;
wherein said second substrate layer is bonded to said first substrate layer upon solidification of said adhesive.

32. A method of making a cosmetic article comprising:
providing a cosmetic composition and an adhesive, wherein at least one of said cosmetic composition and said adhesive is provided in a film;
depositing said adhesive and said cosmetic composition on a first substrate layer;
applying heat to melt said adhesive to provide a melted adhesive; and
superposing a second substrate layer on said first substrate layer and bonding the second substrate layer to said first substrate layer with said melted adhesive.

33. A method of making a cosmetic article comprising:
providing a cosmetic composition powder;
providing an adhesive powder;
depositing said adhesive powder and said cosmetic composition powder on a first substrate layer;
applying heat to melt said adhesive powder to provide a melted adhesive after the adhesive powder and cosmetic composition powder are deposited on said first substrate layer; and
superposing a second substrate layer on said first substrate layer and bonding the second substrate layer to said first substrate layer with said melted adhesive.

34. A method of making a cosmetic article comprising:
depositing an adhesive powder on a first substrate layer;
depositing a cosmetic composition powder on said first substrate layer;
applying heat to heat said adhesive powder to form a melted adhesive; and
superposing a second substrate layer upon said first substrate layer;
wherein said second substrate layer is bonded to said first substrate layer upon cooling of said melted adhesive.
